Europäisches Patentamt

European Patent Office

Office européen des brevets

(11) Publication number: **0 430 443 A1**

## EUROPEAN PATENT APPLICATION

(21) Application number: **90311885.9**

(22) Date of filing: **30.10.90**

(51) Int. Cl.5: **A61F 13/66**

(30) Priority: **08.11.89 US 433235**

(43) Date of publication of application:
**05.06.91 Bulletin 91/23**

(84) Designated Contracting States:
**BE DE ES FR GB IT NL Bulletin**

(71) Applicant: **ARCO CHEMICAL TECHNOLOGY INC.**
**3 Christina Centre Suite 902 201 N Walnut Street**
**Wilmington Delaware 19801(US)**

(72) Inventor: **Gupta, Vijai P.**
**816 Newtown Road**
**Berwyn, Pennsylvania 19312(US)**

(74) Representative: **Cropp, John Anthony David et al**
**MATHYS & SQUIRE 10 Fleet Street**
**London, EC4Y 1AY(GB)**

(54) Disposable liquid-absorbing structure.

(57) Superabsorbent nonwoven web detachably mounted to a structural support material for use as a reusable diaper or incontinence product.

EP 0 430 443 A1

## FIELD OF THE INVENTION

This invention relates to a composite article exhibiting excellent liquid absorption properties. The article of this invention is a nonwoven web of highly absorbent fibers non-permanently attached to a structural support material. Accordingly, the nonwoven web may be combined with a structural support for use as a reusable diaper or incontinence product.

Background of the Invention

Currently, disposable diapers are the diapers of choice for use with infants and young children. However, environmental concerns have been raised in the proper disposal of these diapers. Presently, conventional cloth diapers are the only alternative and offer the convenience of reusing the diaper after washing.

Many conventional disposable products suffer from the disadvantages of having discontinuous areas of absorbent materials as well as problems associated with migration of powder or gel-like particles in the laminate substrate. In addition, the products are deficient in flexibility and other properties.

Accordingly, the present invention provides a composite article exhibiting excellent liquid absorption properties without the shortcomings of conventional disposable products.

Brief Description

According to the present invention, it has been found that superabsorbent articles may be prepared by detachably mounting a superabsorbent nonwoven web to a suitable structural support. Woven, nonwoven or knit fabrics or films and plastic pants may be used as a suitable structural support. The fabrics or films may be cellulosic, olefinic, polyester, acrylic or polyamide fabrics or films, but are not limited to these materials.

It is an object of this invention to provide a composite article comprising a superabsorbent nonwoven web and a structural support material.

It is a further object of this invention to provide a method of making a composite article comprising a nonwoven web with excellent water-absorbing properties and a structural support material.

Detailed Description of the Invention

The invention includes articles and methods for preparing a detachable nonwoven web with excellent water-absorbing properties on a structural support material.

The structural support material comprises woven, nonwoven or knit fabrics or films and includes means for detachably mounting the nonwoven web; via a pocket insert, adhesive, or hook and loop mating for example. Further, the structural support includes plastic pants wherein the nonwoven web is a liner, for example. The preferred location of the nonwoven web is along the ventral to dorsal axis of the structural support. Accordingly, the nonwoven web may be designed as a diaper insert or liner fully lining the interior surface of said structural support. Upon being soiled, the insert or liner is discarded and the structural support may be washed and reused.

The nonwoven web may be used in conjunction with a reusable structural support. The reusable support may be very light weight due to the functionality of the nonwoven web and the nonwoven web may be a thinner absorbent due to the functionality of the structural support. Furthermore, the structural support may be impermeable to liquid.

In another alternative, the nonwoven web may also include an integral light-weight cover-stock as in a disposable diaper for example. The cover-stock is permeable and is disposed on the nonwoven web on the side to be soiled. Accordingly, the cover-stock may be laid over the superabsorbent nonwoven web to form a composite. The exposed side of the nonwoven web is mounted to the structural support. When the nonwoven web is soiled on the cover-stock side, the web-cover-stock is discarded and the structural support is washed and reused.

The nonwoven web may be mounted to the structural support via a pocket; the pocket is optionally made of cover-stock material. The cover-stock pocket is mounted along the ventral to dorsal axis of the structural support and includes a ventral, dorsal or lateral opening. The nonwoven web is inserted into the pocket and removed upon soiling. The cover-stock pocket allows passage of liquid through the pocket and onto the nonwoven web.

Alternatively, the nonwoven web may be mounted to the structural support via adhesive or hook and loop mating. The nonwoven web may be adapted with covered adhesive patches or strips on the exposed side of the support. Upon removal of the covers, the adhesive patches or strips are pressed onto the structural support and removed upon soiling. Nonwoven webs adapted with hook and loop mating would require the structural support to be similarly adapted. Accordingly, the structural support would have a hook in a location compatible with a loop of the nonwoven web. Further, the nonwoven web may line a structural support such as plastic pants.

A typical diaper according to the invention may comprise a "sandwich" of the following layers:
porous polyolefin film
disposable superabsorbent fiber web
cellulosic layer
impermeable polyolefin film

In the above construction, the porous polyolefin film may be a cover-stock, disposed on the side of the diaper to be soiled. The cellulosic layer may be cotton and is optional. The impermeable polyolefin film is also optional and disposed on the exposed side of the diaper. Accordingly, the impermeable polyolefin film layer is disposed on the side lining plastic pants for example.

Nonwoven webs are formed by processes in which fibers are laid down by dispersal in air and air laid by assembly into a sheet in random array. Also, fibers can be laid down as a sheet in a parallel array. The superabsorbent fibers may be prepared by the dry spinning process disclosed in U.S. 4,731,067 or by the air attenuation process of U.S. Patent No. 4,855,179. Alternative fiber-forming methods may be employed as deemed desirable by one skilled in the art.

In one embodiment, the superabsorbent fibers are not used alone, but are air-laid into a web with other compatible fibers. It has been found that fibers which have a density differing by about at least 0.2 g/cc from that of the superabsorbent fibers are surprisingly useful in forming an air-laid web. These webs display a marked gradient in fiber distribution, wherein the higher density superabsorbent fibers are predominantly distributed along one side of the web and the lower density complementary fiber is predominantly distributed along the other side. In moving from one face to the next, there is, therefore, a gradient of superabsorbent fiber, and an associated gradient of absorbent properties.

Aqueous compositions suitable for forming fibers usable in this invention are taught in the following U.S. patents, the teachings of which are incorporated by reference herein: U. S. 4,788,237; U. S. 4,743,244; U. S. 4,705,773; U. S. 4,616,063; U. S. 4,813,945.

A preferred superabsorbent fiber is Fibersorb[R] SA-7000 fiber, available from Arco Chemical Company, Newtown Square, PA. Fibersorb may be used specifically as a nonwoven web. The nonwoven web comprises at least 25% Fibersorb[R] fibers.

While this invention has been disclosed with reference to a specific embodiment, it is apparent that other embodiments and equivalent variations of this invention may be devised by those skilled in the art without departing from the true spirit and scope of the invention. The appended claims are intended to be construed to include all such embodiments and equivalent variations.

## Claims

1. A composite article comprising at least one superabsorbent nonwoven web and a structural support material, wherein said nonwoven web is detachably mounted to said structural support material.

2. An article of claim 1, wherein said nonwoven web comprises at least 25% Fibersorb[R] fibers.

3. An article of claim 1 of claim 2 wherein said nonwoven web is detachably mounted onto the ventral to dorsal axis of said structural support material.

4. An article of claim 1 or claim 2 wherein said nonwoven web is inserted into a pocket of said structural support material.

5. An article of claim 1 or claim 2 wherein said nonwoven web is detachably mounted to said structural support material via hook and loop mating.

6 An article of claim 1 or claim 2 wherein said nonwoven web is detachably mounted to said structural support material via adhesive.

7. An article of any one of claims 1 to 6, wherein said nonwoven web comprises a detchable lining fully covering the interior surface of said structural support material.

8. An article of any one of claims 1 to 7, wherein said nonwoven web has an absorbency gradient.

9. An article of any one of claims 1 to 8 wherein said nonwoven web includes an integral lightweight cover-stock.

10. An article of any one of claims 1 to 9, wherein said structural support material is selected from the group consisting of woven, nonwoven, or knit fabrics or films

11 An article of any one of claims 1 to 9, wherein said structural support material is plastic pants.

12. An article of claim 10, wherein said structural support material is selected from the group consisting of cellulosic, olefinic, polyester, acrylic or polyamide fabrics.

13. An article of claim 10 wherein said structural support material is selected from the group consisting of olefinic, polyester, acrylic, or polyamide films.

14. An article of any one of claims 1 to 13, wherein said structural support is impermeable to liquid

15. An article of any one of claims 1 to 14, comprising a reusable diaper.

16. An article of any one of claims 1 to 14, comprising an incontinence product.

17. A method of making an article of claim 1, comprising detachably mounting said nonwoven web to said structural support material.

18. A method of making an article of claim 1, comprising inserting said nonwoven web into a pocket of said structural support material.

19. A diaper comprising a layered arrangement of a porous polyolefin film, a removable superabsorbent nonwoven web, and an impermeable polyolefin film.

20. A diaper comprising a layered arrangement of a porous polyolefin film, a removable superabsorbent nonwoven web, and a cotton fabric backing.

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| D,X | US-A-4 855 179   (L.G. BOURLAND et al.)<br>* Column 1, lines 6-15; column 7, lines 63-66 *<br>— — — | 1-18 | A 61 F 13/66 |
| Y | | 19-20 | |
| Y | GB-A-2 124 072   (DISPOSABLE DEVELOPMENTS)<br>* Abstract; figure 2 *<br>— — — | 19-20 | |
| A | | 1,3-4,<br>12-13,<br>17-18 | |
| A | EP-A-0 139 351   (E.R. SQUIBB & SONS)<br>* Page 1, lines 7-16; page 4, lines 5-30; page 5, lines 22-26;<br>page 9, line 28 - page 10, line 4 *<br>— — — | 1-3,6-7 | |
| A | GB-A-2 103 930   (VERNON CARUS)<br>* Whole document *<br>— — — | 1,3,6,9,<br>19-20 | |
| A | US-A-4 389 211   (A.R. LENAGHAN)<br>* Column 2, lines 58-64 *<br>— — — | 5 | |
| A | EP-A-0 212 618   (KIMBERLY-CLARK)<br>* Page 6, line 27 - page 9, line 2; page 14, lines 29-31; page<br>15, lines 1-3 *<br>— — — | 2,8,<br>12-13,<br>19-20 | TECHNICAL FIELDS SEARCHED (Int. Cl.5)<br><br>A 61 F<br>A 61 L |
| A | EP-A-0 252 186   (P. VAN MALDEREN)<br>* Column 1, lines 1-13; column 2, lines 1-25; column 2, line<br>50 - column 3, line 9; figure 1 *<br>— — — — — | 12-13,<br>19-20 | |

The present search report has been drawn up for all claims

| Place of search | Date of completion of search | Examiner |
|---|---|---|
| The Hague | 07 March 91 | NICE P.R. |